# EUROPEAN PATENT SPECIFICATION

(11) **EP 4 554 640 B1**
(45) Date of publication and mention of the grant of the patent: **08.04.2026**
(21) Application number: 23748001.7
(22) Date of filing: 11.07.2023
(51) Int. Cl.: A61M 5/32, A61M 5/315, A61M 5/20

(54) **AUTOINJECTOR**
AUTOINJEKTOR
AUTO-INJECTEUR

(30) Priority: 12.07.2022 EP 22184367
(43) Date of publication of application: 21.05.2025
(73) Proprietor: ALK-Abelló A/S, 2970 Hørsholm (DK)
(72) Inventor: MIKKELSEN, Jens, 2970 Hoersholm (DK); CONTERAS MOREIRA, Juan, 2970 Hoersholm (DK)
(74) Representative: Valet Patent Services Limited
(86) International application number: PCT/EP2023/069165
(87) International publication number: WO 2024/013157

(56) References cited:
- US-A1- 2016 193 412
- US-A1- 2020 338 273
- US-A1- 2022 016 356

## Description

### Field of Invention

The present invention relates to an autoinjector.

### Background

Autoinjectors are medical devices for the delivery of medication. In particular, autoinjectors are generally configured for intramuscular or subcutaneous delivery of a self-administration (or untrained administration) of an accurate dose of medication. Autoinjectors may be used for delivery of a range of medications including emergency medications, for example epinephrine for anaphylaxis, or regular medication, for example medication for rheumatoid arthritis. Autoinjectors generally have a multistage actuation sequence which may include a needle penetration step in which a needle is moved to an injection position to cause skin penetration and a dose delivery step in which a piston is actuated to expel medication from a cartridge within the autoinjector. The autoinjector may have a further needle protection stage which makes the device safe after use by shielding the needle (for example by retraction into or deployment of a needle cover). Whilst some autoinjectors may include a user operated button (or other trigger), for ease of operation autoinjectors may be activated by a user grasping a housing of the injector and forcing a front portion of the injector against the injection site. Many such autoinjectors require removal of a safety pin at a rear end to allow for activation and are activated by pressing of a front end located needle shield against an injection site. Such auto have an activation process at opposite ends of the device. This two-ended activation process has potential to cause confusion with some users as to which end to press against an injection site. Some autoinjectors have a safety cap that is removed at a front end, which also breaks a sterility barrier. It is possible that a user may remove the safety cap without meaning to use the device at that time.

US 2016/193412 A1, US 2020/338273 A1, US 2022/016356 A1 disclose examples of the known prior art autoinjectors.

Whilst a variety of autoinjector devices are available there is a desire for improved or alternate autoinjectors which are both easy and/or intuitive for the end user to operate, particularly to avoid foreseeable misuse where a user misunderstands which end is the needle end. There is also a desire for autoinjectors to incorporate safety features which may, for example, prevent accidental operation and/or render the autoinjector safe after use or in the event that a user wishes to return the device to a safe state without use (for example replacing a front end safety cap after removal if the device was not used while sterility is maintained) Further, as autoinjectors are typically single use devices (which require safe disposed as "sharps" clinical waste after use) it is advantageous to ensure that autoinjector devices are designed for efficient manufacture and material usage. Embodiments of the invention seek to address one or more of these needs.

### Summary of Invention

The invention is set out in the appended set of claims.

According to the invention, there is provided an autoinjector, comprising: a main casing; a cartridge assembly disposed within the main casing and including a cartridge and a needle, the cartridge containing a medicament; a power pack disposed within the main casing, the power pack for moving the cartridge and the needle, relative to the main casing, from a storage position to an injection position when actuated; an actuation unit comprising a cartridge container and a needle cover disposed between the cartridge container and the main casing, the actuation unit operably coupled to the power pack and retractable, relative to the main casing, from a first position to an actuation position for actuating the power pack. Thereafter the needle cover is extendable to an extended position covering the needle when the cartridge and the needle are in the injection position. A safety cap is removable from a mounted position on a front end of the main casing to expose a front portion of the actuation unit which includes the needle cover and the cartridge container.

The safety cap is arranged to press a locking arm against the actuation unit when the safety cap is in the mounted position to lock the actuation unit against retraction from the first position until the safety cap is removed. The needle cover includes a through opening and the cartridge container includes a recess so that a front end portion of the locking arm extends through the opening and is pressed by the safety cap into engagement with the recess of the cartridge container.

In particular the safety cap may lock the actuation unit against retraction relative to the casing until the safety cap is removed. The safety cap may lock the actuation unit from moving to the actuation position.

Advantageously, embodiments of the invention provide an arrangement in which the safety cap ensures that the autoinjector is locked against unintended actuation whenever the safety cap is in place on the main casing. This includes for example when the device is initially supplied to a user but also if the user removes and then replaces the safety cap without using the device in the case of a recappable safety cap. In other words, the autoinjector of embodiments has an arrangement which can be made safe even by recapping the safety cap before the device has been actuated. Advantageously, the sterility of the needle / needle assembly is maintained after recapping. That is, the cartridge container, a plunger/piston rod and the cartridge form a sterile enclosure that is not affected by removal of the safety cap.

In embodiments, the actuation unit includes a steril chamber for the needle and removal of the safety cap does not affect sterility of the sterile chamber.

The safety cap may include an inwardly projecting structure, for example a rib, for pressing the locking arm against the actuation unit to lock the actuation unit against retraction from the first position until the safety cap is removed. Advantageously an inwardly projecting structure of the safety cap may engage locking arms radially inwardly of an outer surface of the main casing, as such the locking arms may be somewhat concealed to reduce the risk of a user blocking their movement after removal of the safety cap.

The actuation unit and the locking arm of the main casing may be lockingly engaged when the safety cap is in the mounted position. The actuation unit and the locking arm of the main casing may be free or biased to disengage from locking when the safety cap is removed.

The actuation unit may include a recess or step forming structing in an outer surface thereof. The locking arm may be pressed into engagement with the recess or step forming structure by the safety cap when the safety cap is in the mounted position.

In some embodiments the locking arm is free or biased to splay outwardly out of engagement with the recess or step forming structure when the safety cap is removed.

The front portion of the actuation unit may have a circular cross-section. The safety cap may have a non-circular cross-section, for example an oval cross-section (which may include an elliptical cross-section). That is, the safety cap is shaped to have a main axis in cross-section that is different from a perpendicular minor axis. The cross-sectional shape of the safety cap may generally match the cross-sectional shape of the casing. By providing a circular profile of the front portion of the actuator and a non-matching oval (or similar shape) to the safety cap it is possible to ensure that the opening of the safety cap is always larger than the front portion of the actuation unit regardless of axial orientation of the safety cap. This advantageously reduces the risk of accidental actuation of the device during recapping.

The safety cap and the main casing may include mechanically cooperating features. The features may include a detent to releasably mount the safety cap on the front end of the main housing. An oval (or other non-circular shape) of the safety cap and casing ensures that the cooperating features that engage the locking arm(s) are correctly aligned when the safety cap is recapped.

In some embodiments the safety cap and the front end of the main casing have cooperating oval cross-sections. The cooperating sections may provide for a slide fit mounting and recapping of the safety cap and the front end of the main casing.

The safety cap may be configured to be removable from the front end of the main casing by translation without rotation. Visual and/or tactile features may be provided on the exterior of the cap to indicate the removal action of the safety cap.

In some embodiments the autoinjector may comprise first and second locking arms that are arranged at diametrically opposed positions. As herein the safety cap is arranged to press the first and second locking arms against the actuation unit when the safety cap is in the mounted position to lock the actuation unit against retraction from the retracted position until the safety cap is removed.

In some embodiments the locking arm has a front end that is enlarged to project inwardly.

In embodiments the main casing comprises a window opening. The main casing may further comprise a transparent window piece inserted into the main casing to close the window opening. A forwardly projecting locking arm may be formed integrally with the window piece. The main casing may comprise a pair of diametrically opposed window openings. A transparent window piece may be inserted into each window opening and each window piece may comprise a forwardly projecting locking arm.

Unless otherwise stated, each of the integers described may be used in combination with any other integer as would be understood by the person skilled in the art. Further, although all aspects of the invention preferably "comprise" the features described in relation to that aspect, it is specifically envisaged that they may "consist" or "consist essentially" of those features outlined in the claims. In addition, all terms, unless specifically defined herein, are intended to be given their commonly understood meaning in the art.

Whilst the invention has been described above, it extends to any inventive combination of the features set out above or in the following description or drawings.

### Description of the Drawings

Embodiments of the invention may be performed in various ways, and embodiments thereof will now be described by way of example only, reference being made to the accompanying drawings, in which:
Figure 1 shows an exploded view of an autoinjector in accordance with an embodiment;
**Figures 2A** **and** **2B** shows orthogonal side and cross-sectional views of the autoinjector of Figure 1 in an initial, pre-use, state;
**Figure 3A** **and** **3B** shows orthogonal side and cross-sectional views of the autoinjector of Figure 1 in a ready-to-actuate state;
**Figure 4A** **and** **4B** shows orthogonal side and cross-sectional views of the autoinjector of Figure 1 in a fired state;
**Figure 5A** **and** **5B** shows orthogonal side and cross-sectional views of the autoinjector of Figure 1 in a final, post-use, state;
**Figure 6** shows isolated views of a power pack for use in the autoinjector of Figure 1 in an initial, pre-use, state;
**Figure 7** shows isolated views of the power pack of figure 6 in a ready state;
**Figure 8** shows isolated views of the power pack of figure 6 in a depressed/triggered state;
**Figure 9** shows isolated views of the power pack of figure 6 in a fired state;
**Figure 10** shows isolated views of the power pack and actuation unit of the autoinjector of figure 1in an initial, pre-use, state;
**Figure 11** shows the power pack and actuation unit of figure 10 in a depressed/triggered state;
**Figure 12** shows the power pack and actuation unit of figure 10 in a final, post use, state;
**Figure 13** shows a cross sectional detail of the autoinj ector of Figure 1;
**Figure 14A to 14D** show a range of piston rods for use in autoinjectors of embodiments;
**Figure 15** shows an alternate configuration of a safety cap in accordance with embodiments;
**Figure 16** shows an alternate configuration of a main casing in accordance with embodiments;
**Figure 17** shows an alternate configuration for the actuation unit and power unit for use in embodiments;
**Figure 18A and 18B** shows an alternate configuration for the cartridge sleeve for use in embodiments; and
**Figure 19A to D** show the labelling arrangement for use in an embodiment.

### Detail Description of Embodiments

It may be noted that forward and rearward are used herein to conveniently refer to aspects of the device with reference to its typical in use orientation. Thus, it will be understood that forward may generally mean a surface, component or direction which is proximal to or facing the medicament delivery end (e.g. needle end) of the device. Likewise, it will be understood that rearward may generally refer to a surface, component or direction distal to the delivery end. However, it will be appreciated that such references are not intended to be limiting and that the device may take any orientation in use. References to circumferential, radial or axial directions may also be used herein to as generally geometric terms of orientation with respect to the longitudinal axis of the autoinjector device (which will typically coincide with the longitudinal direction towards the needle end). However, such terms are not intended to be interpreted in a narrow or limiting manner and, for example, do not exclude that a component may have a non-circular or irregular form.

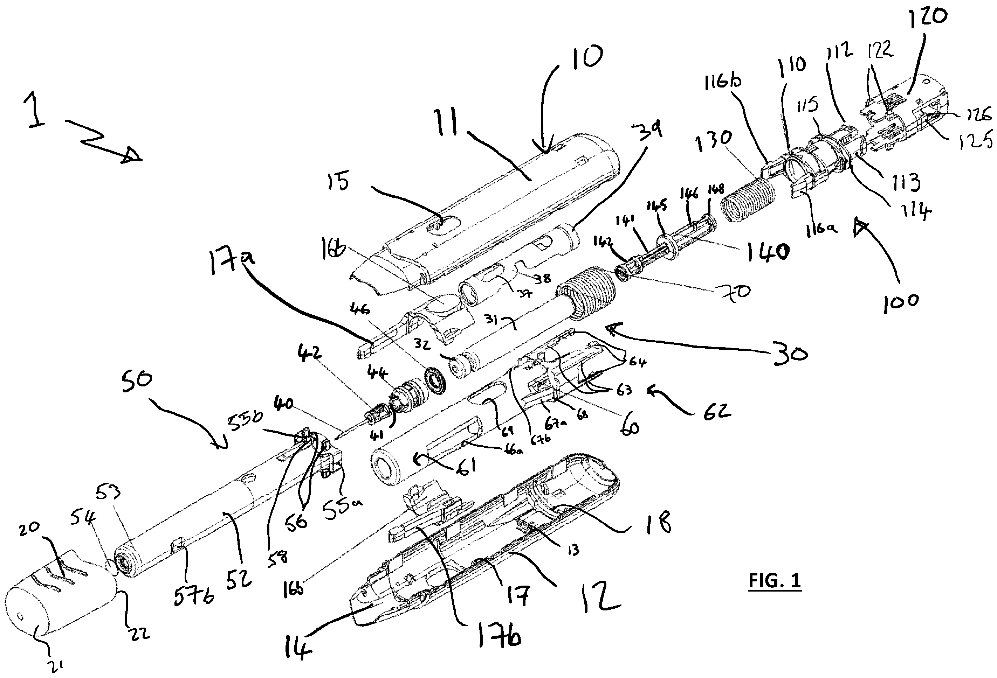

An autoinjector 1 in accordance with an embodiment of the invention is shown in Figures 1 to 5. The autoinjector 1 comprises a main casing 10 closed by a cap 20 (also named safety cap in here). Within the main casing 10 the autoinjector comprises a cartridge assembly 30, an actuation unit 50 and a power pack 100, which includes a spring 130. The actuation unit 50 includes a cartridge container 52, a needle cover 60 and a needle cover spring 70. The needle cover 60 is disposed radially between the cartridge container 52 and the main casing 10.

The auto injector 1 includes a main casing 10 which can be formed from two body shells 11 and 12, which are configured to resiliently snap-fit together during assembly of the device. The snap fit connection will typically be an irreversible engagement such that an end-user cannot disassemble the casing 10 and cannot therefore access the internal components of the autoinjector 1. A label (discussed further below) locks the two body shells 11, 12 together by bridging a join between then, providing added strength and making the body shells 11, 12 more difficult to disassemble. Advantageously, therefore, the autoinjector might be supplied without the need of a protective case or carry case. The main casing 10 has an elongate tubular profile with a closed rear end and an opening 14 at the forward end. Having a closed rear end helps ensure that the autoinjector is intuitive to use since the user can immediately identify which end of the device is operated from. The cross-sectional profile of the casing 10 is generally oval. It may be appreciated that oval is used broadly herein and may include an elliptical or superelliptical cross-section and may allow for attaching a label to the main casing The oval shape advantageously prevents rolling and thus accidental falling from a surface.

The main casing 10 may be formed from an injection moulded plastic material. The main casing may be opaque. One or more windows 15 may be formed in sides of the casing 10, for example in the form of through openings. A transparent window piece 16 may be inserted into the main casing 10 to close the window openings. In the illustrated embodiment the window piece has an annular form and comprises two half sections 16a, 16b each of which interconnects with a respective body shell 11, 12 of the main casing 10. The window pieces also each include a forwardly projecting locking arm 17a, 17b which will be described further below. A safety cap 20 (described in further detail below) is removably mounted on a front end of the main casing 10 and closes the front opening 14.

As will be explained below with reference to the corresponding features, the internal surfaces of the main casing 10 may be provided with integrally moulded features which project inwardly into the autoinjector 1 to provide cooperating structures for components of the actuation unit 50 or power pack 100. The features may for example include ribs 17, flanges 18 and/or stops 13.

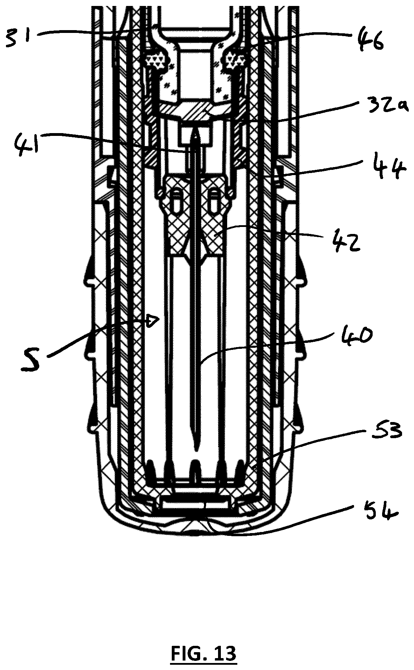

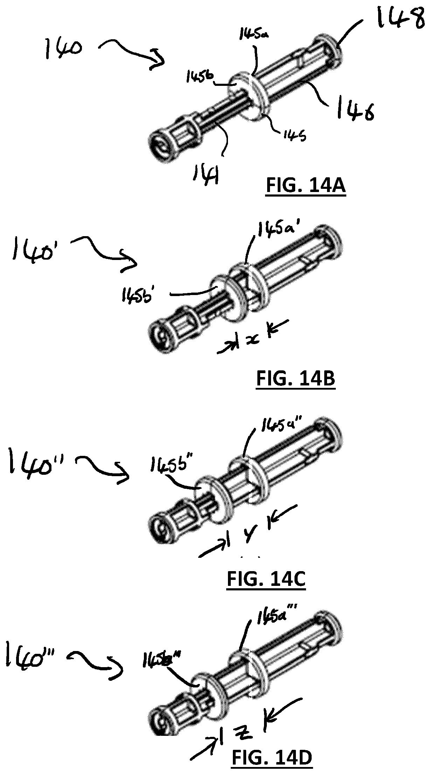

A cartridge assembly 30 is disposed within the main casing 10. The cartridge assembly supports a cartridge 31. The cartridge 31 is typically a glass container of medicament with a stopper 35 (not shown in figure 1) at a rear end which is movable to dispense medicament out of the cartridge. The size of the cartridge 31 may be selected based upon the dosage or medication the autoinjector is intended to deliver, and may for example be in the range of 0.3 mL to 2.5 mL, such as approximately 1mL, or 1.5 mL. As will be described further below with reference to figure 14, in some embodiments, a single size of cartridge 31 may be used in several autoinjector configurations which deliver a different volume of the same medicament and thus provides the option to deliver a different single dose in use (with more volume of the medicament being left in the cartridge after use when the delivery volume is lower). The forward end of the cartridge 31 may be closed with an aluminium crimp cap 32 with a septum 32a (see figure 13), for example a bromobutyl septum, which seals the medicament inside the cartridge. The septum 32a is pierceable allowing for the penetration of a needle.

Forward of the cartridge 31, the cartridge assembly 30 includes a needle 40 which is carried by a needle guide 42. A detailed section showing the needle 40 is shown in figure 13. A rear end of the needle 40 projects beyond the needle guide 42 towards the septum 32a of the cartridge 31. The needle guide 42 is aligned with respect to the forward end of the cartridge 31 by a guidance sleeve 44 which is sized to sit on the shoulder of the crimp camp 32 of the cartridge 31. A sealing ring 46 is positioned between the guidance sleeve 44 and the cartridge 31 (and can pass over the crimp camp 32 during assembly of the autoinjector to be seated on a neck of the cartridge 31).

The cartridge assembly 30 also includes a cartridge sleeve 38 which is slid around the cartridge 31 during assembly. The rearward end of the cartridge sleeve 38 is provided with a lip 39 which seats on the rear (open) end of the cartridge 31. The lip 39 defines an opening at the rear of the cartridge assembly which is of reduced diameter in comparison to the internal diameter of the cartridge 31. The cartridge sleeve 38 is also provided with windows 37 in the form of through openings so as to avoid obstructing the view of the contents of the cartridge 31. The cartridge sleeve 38 is axially and rotationally connected to the piston rod 140 and can thus not rotate and obscure the window. The piston rod 140 can move forward but not rearward.

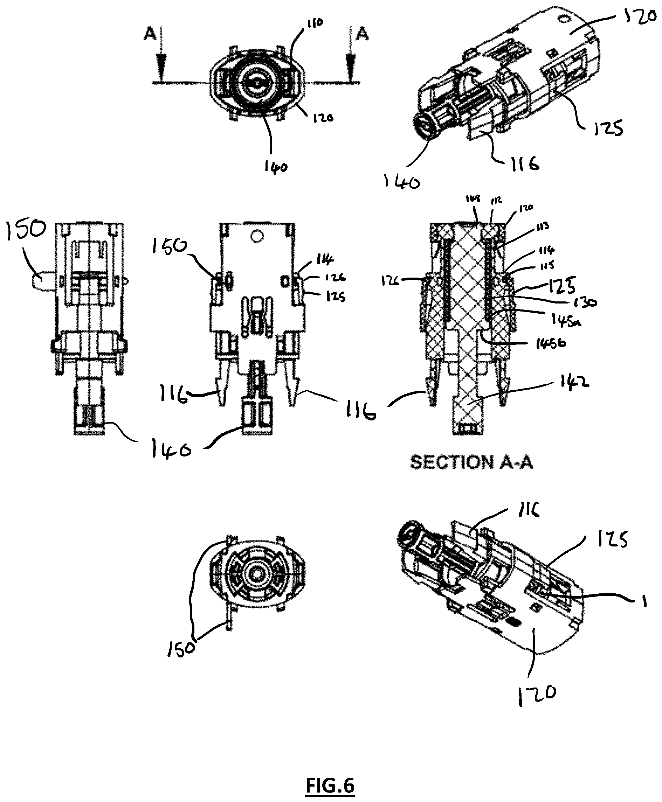

A power pack 100 is also disposed within the main casing 10 for moving the cartridge assembly 30 relative to the main casing 10 from a storage position to an injection position when actuated (as will be explained below). The power pack 100 includes an inner body 110, an outer body 120, a spring 130 and a piston rod 140. The spring 130 stores energy to actuate the autoinjector 1 and when released is used to drive the piston rod 140 and the cartridge assembly 30 to an actuation position. The inner body 110 and outer body 120 are configured to hold the spring 130 in a latched state and release the spring to activate the device. The assembled power pack 100 is shown in isolation and in various stages of operation in Figures 6 to 8.

The piston rod 140 is an integrally moulded part. The piston rod 140 has a forward portion 141 which is configured to extend through a rear opening of the cartridge 31 and engage the piston 35. The engaging end of the forward portion 141 may have an enlarged or bulbous head 142 for engaging the piston 35. The lip 39 of the cartridge sleeve 38 has a smaller internal diameter opening than the outer diameter of the head 142 of the piston rod. As such, the lip 39 engages the rearward end of the head 142 and maintains connection between the cartridge 31 and the piston rod 140. A rearward portion 146 of the piston rod 140 is circumferentially surrounded by the spring 130 (and the spring 130 and piston rod 140 are generally coaxial). A flange 145 extends around a midportion of the piston rod 140 and defines a separation between the forward 141 and rearward 146 portions. A rearward face of the flange 145 provides a spring shoulder against which a forward end of the spring 130 is mounted. The rear end of the piston rod 140 includes a latch head 148 which is engageable by the inner body 110 of the power pack 100 (as will be described further below).

The inner body 110 is a substantially tubular integrally moulded part which generally surrounds the piston rod 140 and the spring 130 (the spring being radially intermediate to the rod and inner body). The forward end of the inner body 110 includes latch members 116a, 116b which provide snap-fit assembly features between the power pack 100 and the actuation unit 50. The rearward end of the inner body 110 comprises a collet 112. The collet 112 comprises four collet arms 113 circumferentially distributed around the inner body 110 and each being a segment of the collet 112. Each collet arm 113 extends rearwardly from a collar 114. The collar 114 has an oval cross section which generally conforms to the shape of the autoinjector 1 as defined by the main casing 10 (and supports and aligns the outer body 120 relative to the inner body 110). The collar 114 includes a recess 115 which is configured to receive and retain the heads 126 of latch arms 125 (described further below) of the outer body 120.

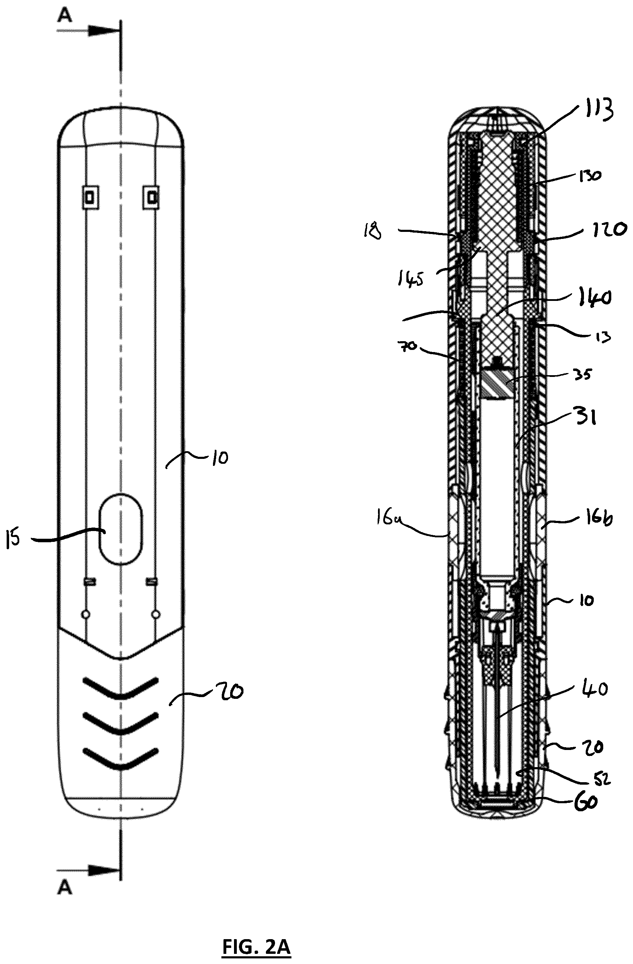

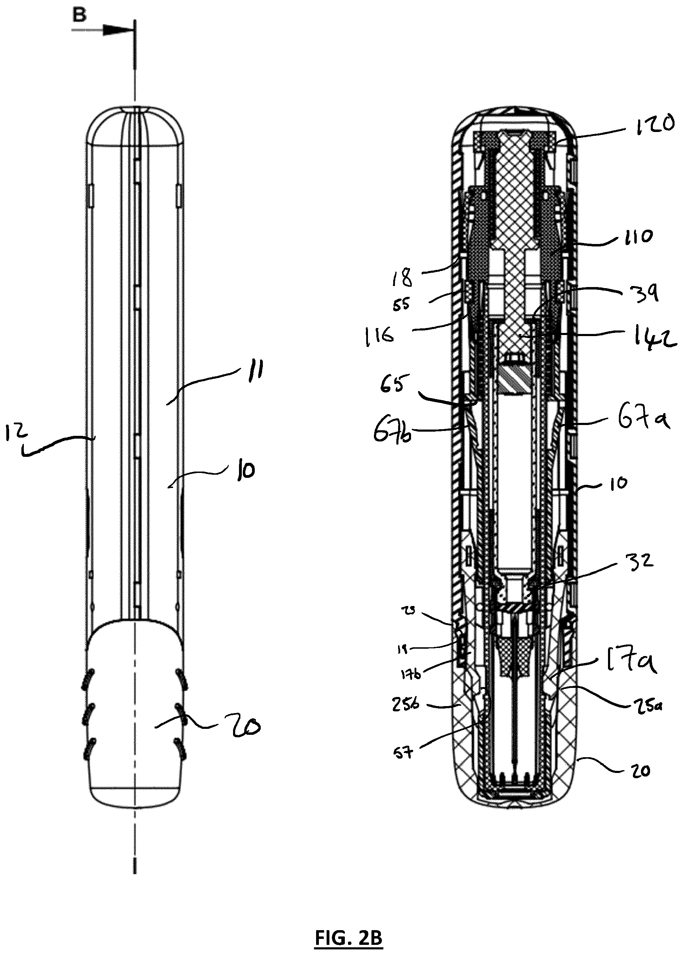

As best seen in figures 2 and 6, the collet 112 of the inner body 110 is shaped and configured to engage and retain the latch head 148 of the piston rod 140. Specifically, the rearward ends of each collet arm 113 includes a radially inwardly extending engagement surface which, in the initial state of the device mate with a forward shoulder of the latch head 148. It may be noted from the cross-sectional view of figure 6, that the shoulder of the latch head 148 and engagement surfaces of the collet arms 113 are provided with respective cooperating sloped surfaces 148a and 113a. Specifically, the sloped profiles of the cooperating surfaces 148a and 113a are forwardly inwardly angled such that (as will be described further below) the latch head 148 can slide forward through the collet 112 camming the collet arms 113 outwardly. The rearward ends of the collet arms 113 further define an annular forward-facing surface on the underside of the collet 112. The forward-facing surface provides a spring shoulder against which a rearward end of the spring 130 is mounted. In the illustrated embodiment, there are four collet arms 113 where two collet arms 113 support the piston rod 140 and are able to release the piston rod when the collet is moved whilst the other two collet arms 113provide the spring should for the spring 130.

The outer body 120 is a generally oval tubular body and circumferentially surrounds the inner body 110 (such that it is intermediate between the inner body and main casing). The outer body 120 may be considered a trigger member, which maintains the engagement between the collet 112 and piston rod 140 in an initial position against the bias of the spring 130. As will be explained below, relative axial movement between the inner body 110 and outer body 120 can move the outer body 120 out of alignment with the collet arms 113 to enable the collet to disengage. As the outer body 120 is generally fixed relative to the main casing 10, the outer body 110 and the main casing 10 are provided with interconnecting geometry to provide a positive location therebetween. For example, in the illustrated embodiment the main casing 10 includes an internal flange 18 which the forward end of the outer body 120 abuts when the autoinjector is assembled.

The forward end of the outer body 120 includes four radially outwardly extending tabs 122. The tabs 122 are arranged in two pairs on opposing sides of the outer body 120, the tabs of each pair being circumferentially spaced apart. As will be explained below, the tabs 122 provide an interaction surface for the needle cover 60 to add to the robustness of the autoinjector and ensure accurate sequencing of the spring release. The tabs 122 further act as push rods to release the needle cover 60 in that when the needle cover locking latches 64 (also termed hooks herein) are pressed against them, the arms 63 of the locking latches bend out and releases the grip on towers 56 (also termed bosses herein) on the cartridge container 56

The outer body 120 also includes two rearwardly extending latch arms 125. The latch arms 125 are diametrically opposed. Each latch arm 125 is a flexible axially elongate member which is integrally formed with the outer body 120. The latch arms extend from a forward end which is contiguous with the outer body 120 to a free rearward end which includes an inwardly projecting head 126.

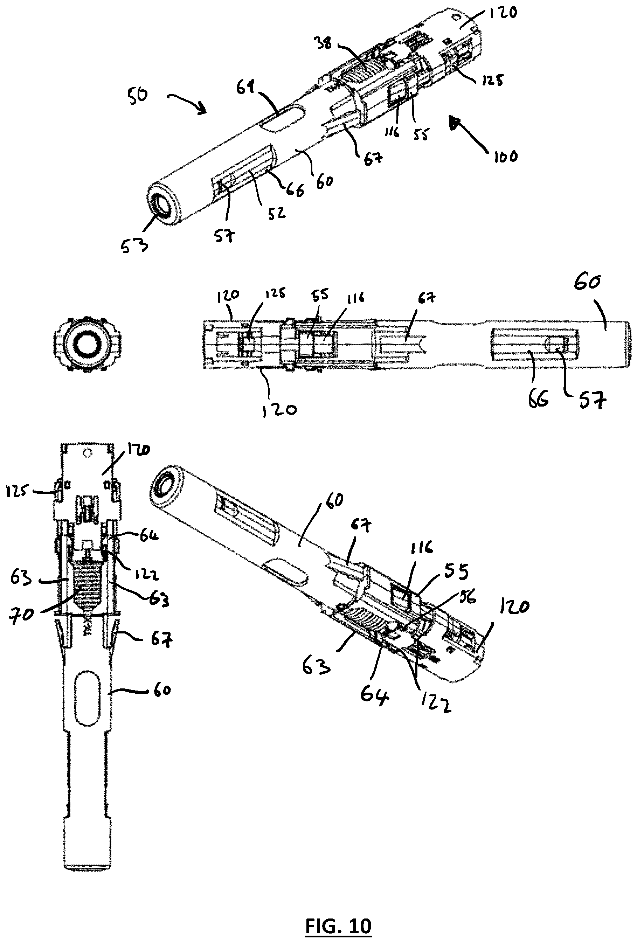

The autoinjector 1 further comprises an actuation unit 50 comprising a cartridge container 52, a needle cover 60 and a needle cover spring 70. Figures 10 to 12 show isolated views of the actuation unit 50 and power pack 100 in various stages of operation. The cartridge container 52 is a generally tubular body which is configured to enclose the cartridge 31 and the cartridge assembly 30 (which includes the needle assembly 40). The forward end of the cartridge container 52 includes an aperture 53 which is initially closed by a membrane 54. The cartridge assembly 30 is inserted into the cartridge container 52 from an opening at the rearward end. As best seen in the detailed section of figure 13, the sealing ring 46 of the cartridge assembly 30 can provide a seal between the external surface of the cartridge 31 and the internal surface of the cartridge container 52. By being positioned around the neck of the cartridge 31 the sealing ring 46 both provides a compact arrangement and also ensures the correct (co-axial) positioning of the front end of the cartridge (assisted by the guidance sleeve 44) within the cartridge container 52.

The forward portion of the cartridge container 52 can form a chamber (S in figure 13) extending axially from the membrane 54 to the sealing ring 46. Prior to activation of the autoinjector 1, the needle assembly is fully contained within the chamber S to maintain sterility of the needle assembly 40. The chamber S may have a gas permeable opening - for example one or more of the membrane 54, the sealing ring 46 or a dedicated orifice may include a gas permeable material. With the sealed chamber S closed by a porous material bacteria, viruses or fungi cannot enter the area around the needle assembly 40. However, the material is not a barrier to gas (including atmospheric gasses). This arrangement allows the assembly to be sterilised during manufacture using a gas such as Ethylene Oxide. Whilst the illustrated embodiment uses a gas permeable membrane 54, other embodiments may provide alternative ways to maintain the sterile barrier in the cartridge container 52.

The rearmost portion of the cartridge container 52 is provided with interface to enable interconnection between the actuation unit 50 and the power pack 100. This interconnection can be best seen in figures 10 to 12. In particular, the cartridge container 52 is provided with a pair of slots 55a and 55b which are configured to receive the latch members 116a, 116b of the inner body 110 of the power pack 100. The slots 55a, 55b include a forward end with a shoulder, which the latch features 116 can snap beyond to join the power pack 100 and actuation unit 50. When the latch members 116 snap into place in the slots 55, the needle cover spring 70 is axially between the inner side of the latches on the inner body 110 and the cartridge container 52 (see for example figures 10 to 12). As such, it may be noted that the needle cover spring 70 underlies the ends of the latch members 116. This arrangement advantageously supports the latched configuration of the latch 116 and slots 55 and blocks the latch members 116 from deflecting out of engagement once the autoinjector 1 has been assembled (and therefore enhances the robustness of the assembled connection between the power pack 100 and actuation unit 50).

The rear end of the cartridge container 52 also includes radially outwardly projecting bosses 56. The bosses 56 are arranged in two pairs on opposing sides of the cartridge container 52, the bosses of each pair being circumferentially spaced apart. As will be explained below, the bosses provide a catch for connection between the cartridge container 52 and needle cover 60. Adjacent to the bosses 56 there is also provided a forward-facing seat 58 for receiving the rearward end of a needle cover spring 70.

A forward portion of the cartridge container 52 includes a pair of diametrically opposed external recesses 57a and 57b (or other step-forming features). The recesses are sized and shaped to receive and be engaged by the enlarged inwardly projecting free ends of the forwardly projecting locking arms 17a, 17b of the main casing 10. As will be described further below, in the initial configuration of the device the recesses 57a, 57b (of which only 57b is visible in figure 1) and ends of locking arms 17a, 17b are axially aligned.

The needle cover 60 is also a substantially tubular member and externally surrounds the cartridge container 52 such that the needle cover is disposed between the cartridge container 52 and the main casing 10. The needle cover comprises a forward portion 61 which closely conforms, with a small clearance, to the external profile of the cartridge container 52 and a rearward portion 62 which has an increased cross-sectional area. The profile of the rearward portion 62 provides an annular cavity around the cartridge container 52 which accommodates the needle cover spring 70 such that the spring is contained between the cartridge container 52 and needle cover 60. The interface between the rearward portion 62 and forward portion 61 of the needle cover 60 may include a step change in profile such that a rearwardly facing seat 65 is defined for the forward end of the needle cover spring 70.

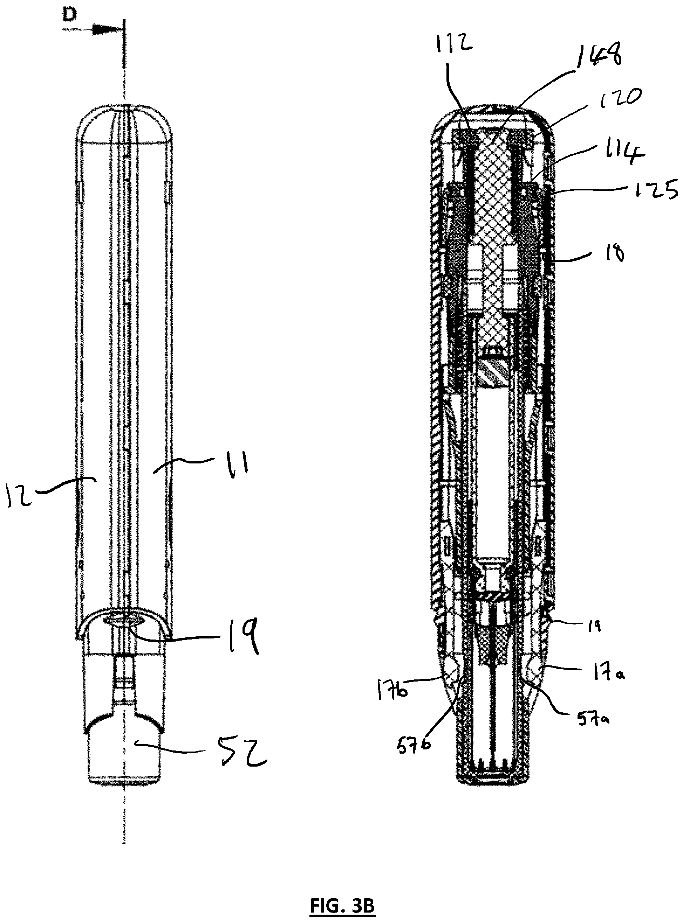

Integrally formed with the rearward portion 62, the needle cover 60 includes four hooks 64 each formed at the end of a resilient rearwardly extending arm 63. As will be explained further below, the hooks 64 form a first locking assembly which in an initial configuration engage the bosses 56 of the cartridge container 52 to form a catch between the needle cover 60 and the cartridge container 52. The catch holds the needle cover in a first position against the forward bias of the needle cover spring 70. The needle cover spring 70 is initially compressed between the rearward facing seat 65 of the needle cover 60 and the forward-facing seat 58 of the cartridge container 52. It may also be noted (particularly in the cross-sectional views of figure 2A and 3B) that the rear end of the needle cover spring is also supported by the inwardly projecting stops 13 of the main casing 10. In the initial (pre-use) configuration of the autoinjector 1 the stops 13 and the forward facing seat 58 are longitudinally aligned.

The needle cover 60 also includes a second locking assembly comprising a pair of diametrically opposed outwardly extending resilient members 67a and 67b. Each resilient member 67 is integrally formed at the rearward part of the forward portion 61 of the needle cover 60. The resilient members project outwardly and rearwardly from a forward end which is integral with the needle cover 61 to a free end 68. As will be explained further below, as the resilient members 67 are angled rearwardly they can cam inwardly during forward movement to elastically deflect the free end 68 inwardly over a feature such as a ledge. In contrast, in rearward movement the free end 68 of the resilient member will engage features such as a ledge and will lock the needle cover 60.

The forward portion 61 of the needle cover 60 includes a pair of diametrically opposed through openings 66a, 66b. The openings 66a and 66b are axially and circumferentially aligned with the recesses 57a and 57b of the cartridge container 52. As such, the locking arms 17a, 17b of the main casing 10 may each extend through a respective one of the through openings 66a, 66b to engage their respective recess 57a, 57b.

The forward portion 61 of the needle cover 60 also includes a window 69 (typically a pair of diametrically opposed windows), which may be a through opening. As the window 69 is in the forward portion 61 of the needle cover 60 it is forward of the needle cover spring 70. In the initial configuration of the autoinjector, the window 69 of the needle cover 60 is axially and circumferentially aligned (or at least partially overlapping) with the window 15 of the main casing 10.

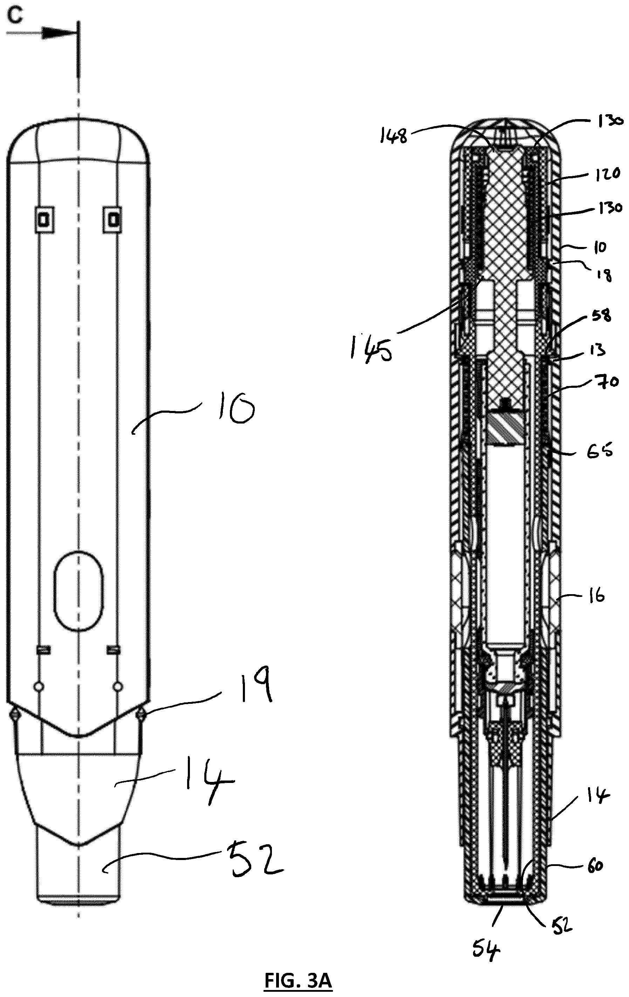

The cap 20 has an oval cross section and a cup shaped form with a closed front end 21 and an open rear 22. Externally the cap 20 may be provided with one or more tactile features to make the cap easily and intuitively graspable by a user. Advantageously, in the illustrated embodiment the tactile features are three chevron shaped ridges which both provide additional gip and indicate the direction and action required to remove the safety cap 20. The oval cross section of the safety cap has a complementary shape to the tapered front end of the main casing 10. The cap 20 and the main casing 10 are configured to provide a slide fit which enables mounting and recapping of the cap 10 and the front end of the main casing 10 to cover the front opening 14. As best seen in figures 2B, 3A and 3B, a detent is provided to releasably mount the safety cap 20 on the front end of the main housing 10 - for example the internal surface of the cap 20 may include projections 23 which engage corresponding features 19 (as shown in figure 2B) in the external surface of the main casing 10.

Internally the cap 20 is configured to cooperate with the outer surface of the locking arms 17a, 17b of the main casing 10. In particular, the cap 20 may include inwardly projecting features such as ribs 25a, 25b which press the locking arms 17a and 17b radially inwardly against the actuation unit 50 such that the ends of the arms extend through openings 66a, 66b of the needle cover 60 and to engage the respective recess 57a, 57b of the cartridge container 52. As such, when the cap 20 is in position (e.g. mounted) on the outer casing 10 the actuation unit 50 is locked in position relative to the outer casing 10.

Operation of the autoinjector 1 will now be described. The initial configuration (e.g. the configuration before use, such as delivered to the end-user) of the autoinjector pen is shown in Figure 2 in which the safety cap 20 is attached to the forward end of the outer casing 10. The corresponding configuration of the power pack 100 is shown in Figure 6 and the actuation unit 50 and power pack 100 in Figure 10. In this configuration the ribs 25 of the cap 20 are forcing the locking arms 17 inward to lock the activation unit 50 relative to the casing 10. The needle cover 60 is latched to the power unit 100 by the engagement of the first locking mechanism (the hooks 64 engaging the bosses 56 of the cartridge container 52). In this position, the needle cover spring 70 is held in a compressed state with the forward end on the rearward facing seat 65 of the needle cover 60 and the rearward end against both the seat 58 of the cartridge container and the stops 13 of the main casing 10.

As best seen in figure 6, the power pack 100 (specifically the inner body 110 thereof) is also initially in a latched configuration and held against the forward bias of the spring 130. The latched state is maintained by the latch head 148 of the piston rod 140 being held in the collet 112 of the inner body 110. The outer body 120 of the power pack 100 externally surrounds the collet 112 to block radial expansion thereof (which would be required to release the head 148 of the piston rod 140).

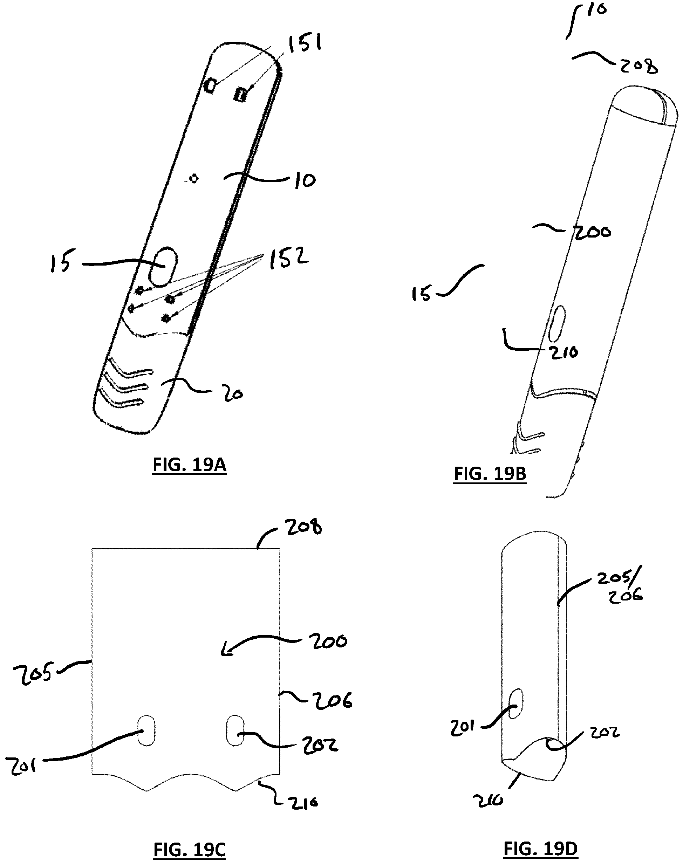

It can be noted that the power pack 100 may also be provided with a safety pin 150 (as shown in Figure 6) which mechanically blocks movement between the inner body 110 and outer body 120. The safety pin 150 can be inserted through an appropriate through-aperture (see for example 151 in Fig 19A) in the main casing 10. Such a safety pin may for example be used to ensure that the power pack cannot be accidentally released during assembly of the autoinjector 1. Once the autoinjector 1 is fully assembled (with the cap 20 attached) the safety pin 150 is no longer needed to isolate the power pack. As such, the safety pin may be removed in one of the final assembly steps and prior to packaging and delivery of the autoinjector to an end user. The label 200 (discussed further below) the hole through which the safety pin 150 extended to provide ingress protection and structural robustness.

In the initial configuration, the window 15 of the outer casing 10, the window 69 of the needle cover 60, and the window 37 of the cartridge sleeve 38 are aligned whilst the cartridge container 52 is made at least partly of a transparent material. As such, the user has an unimpeded view and may visually inspect the medicament in the cartridge 31 through the aligned windows 15, 69, 37 and an aligned transparent area of the cartridge container 52.

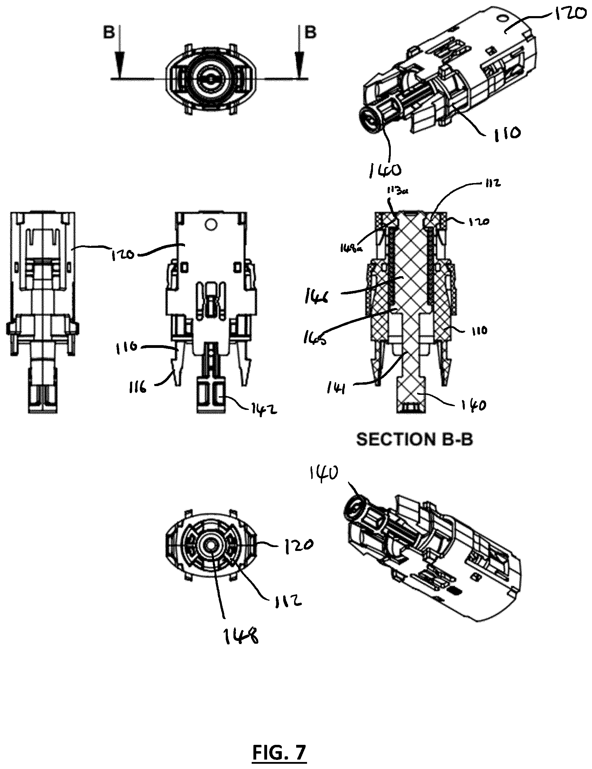

When use of the autoinjector 1 is required, the user removes the cap 20 by pulling it axially away from the main casing 10. The detent features 19, 23 of the main casing 19 and cap 20 provides mild resistance but can be easily overcome by the user. The ready to use configuration of the device is shown in Figure 3 and Figure 7 shows the corresponding state of the power pack. It can be noted that with the cap 20 removed, the forward end of the actuation unit 50 (which includes the forward portions of both the needle cover 60 and the cartridge container 52) extends forwardly of the opening 14 of the main casing 10. In this initial state, it may be noted that the needle assembly 40 is positioned such that the rearward needle tip is forward of the septum 32a, and the forward needle tip is rearward of the membrane such that it is fully within the sterile chamber defined in the cartridge container 52.

With the cap 20 removed, the locking arms 17a and 17b are no longer held inwardly and can resiliently splay outward such that the ends move out of engagement with the recesses 57a, 57b of the cartridge container 52. By that the cartridge container is released and can be moved rearwards to release the spring.

If the user chooses not to activate the autoinjector 1 the removal of the safety cap 20 from the casing 10 can be reversed with the safety cap 20 re-positioned onto the autoinjector. Such recapping of the autoinjector 1 positions the ribs 25a, 25b back into engagement with the locking arms 17a and 17b such that they engage the recesses 57a, 57b. Thus, recapping will render the autoinjector safe and locks the actuation unit 50 in position relative to the outer casing 10. A recapping capability of the safety cap 20 is optional. Further as the needle assembly 40 does not leave the sterile chamber S during removal or replacement of the cap 20, sterility of the device is maintained if a user uncaps the device without subsequent actuation.

To activate the autoinjector 1, the user grasps the main casing 10 and presses the front end of the device (the forward end of the actuation unit 50, e.g. the front end of the cartridge container/needle cover) against an injection site. The force on the front end causes rearward movement of the actuation unit 50 relative to the main casing 10. The locking arms 17a, 17b of the main casing 10 are no longer inwardly restrained and can deflect outwardly so do not prevent the relative movement. The cartridge container 52, needle cover 60 and needle cover spring 70 of the actuation unit are all moved rearwardly.

During initial rearward movement of the actuation unit 50 the seat 58 of the cartridge container is moved rearwardly away from the stops 13 on the main casing 10. The seat 65 at the front of the needle cover 60 moves longitudinally towards the stops 13. The rearward end of the needle cover spring 70 is unable to move past the stops 13 and is disengaged from the seat 58. Accordingly, the movement of the actuation unit 50 results in a further compression of the needle cover spring 70 between the seat 65 and stops 13. Advantageously, this configuration helps to provide the autoinjector with a reliable activation force (for example between 40 to 60% of the force may be from the spring).

Additionally, the compression of the needle cover spring 70 may be used to ensure that the autoinjector returns to a safe pre-use position if not activated (and can therefore be safely recapped). Specifically, if a user only carries out a partial activation movement (i.e. they do not press the autoinjector 1 sufficiently to move the actuation unit 50 all the way to the activation position in which drug delivery is initiated) before removing pressure on the device the needle cover spring 70 will to act to return the actuation unit 50 to its pre-use or storage configuration by biasing the actuation unit 50 forward relative to the main casing 10 until the stops 13 and seat 58 are realigned.

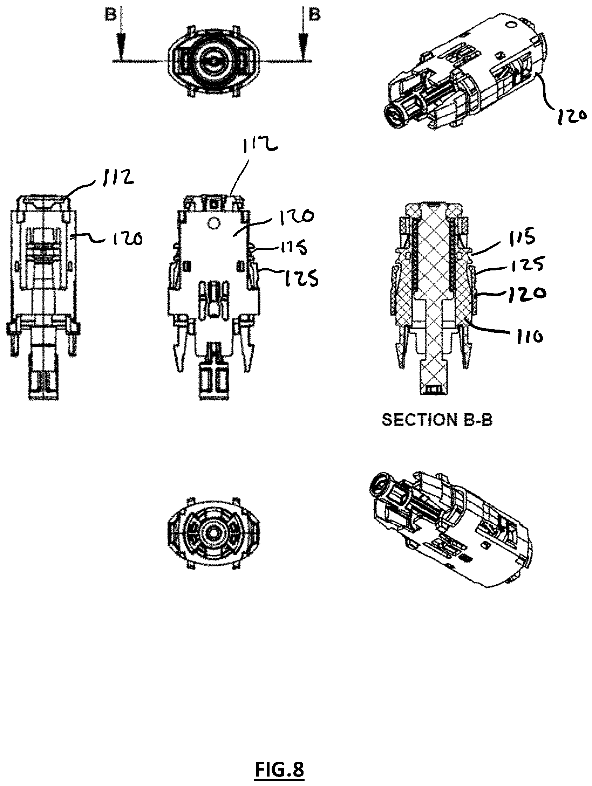

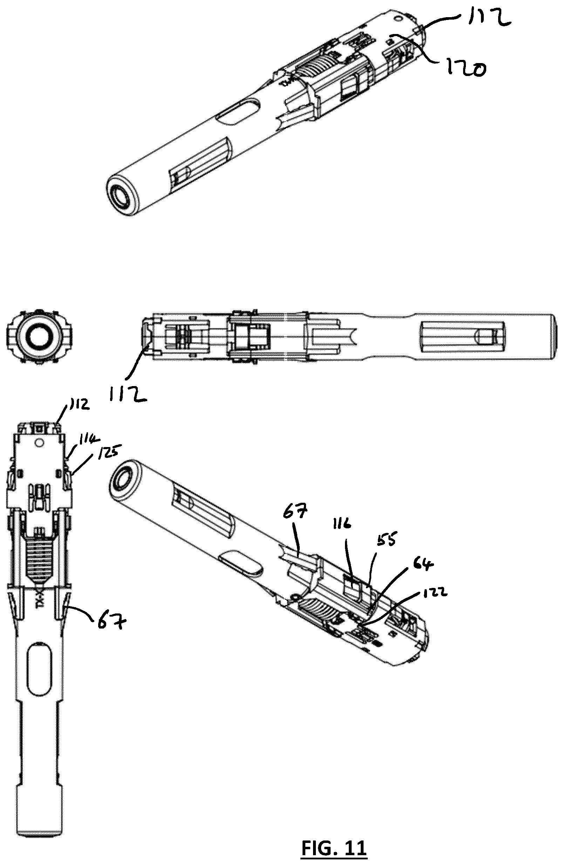

Due to the connection between the inner body 110 of the power unit 100 and the cartridge container 52, the inner body of the power unit 100 also moves relatively rearwardly with the actuation unit 50. The inner body 110 moves as a unit with the piston rod 140 and spring 130. The outer body 120 of the power unit 100 is fixed relative to the main casing 10. The resulting relative movement between the outer body 120 and inner body 110 causes the collet 112 of the inner body 110 to move rearwardly out of alignment with the surrounding portion of the outer body 120. This can be clearly seen in the isolated views of figures 8 and 11 in which the inner body 110 extends rearwardly beyond the outer body 120.

As a result of the rearward movement, the collet 112 is no longer restricted from expanding radially outwardly. Due to the forward force of the spring 130, the cooperating surfaces 148a of the latch head 148 are forced into bearing on the corresponding cooperating surfaces 113a of the collet arms 113. The slope of the cooperating surfaces 148a and 113a acts to cam the collet arms 113 radially outwardly and release the latch head 148 of the piston rod 140. This enables the spring 130 to freely act and begin urging the piston rod 140 forwards. In conjunction with this release of the piston rod 140, it can be noted that the heads 126 of the latch arms 125 of the outer body 120 move out of the recess 115 in the collar 114 of the inner body. This enables the latch arms 125 to snap inwardly in front of the collar 114. As such, the triggered configuration of the power unit 100 is maintained even if the user does not continue to maintain pressure on the main casing 10 of the autoinjector 1.

During activation, the relative rearward movement of the needle cover 60 also causes the release of the first locking assembly. The hooks 64 of the needle cover 60 are pushed into engagement with the tabs 122 of the outer body 120. This causes the resilient arms 63 carrying the hooks 64 to be resiliently deflected transversely such that they move out of engagement with the bosses 56. This results in the needle cover 60 being unlocked and ready to deploy under the forward force of needle cover spring 70. However, at this stage the needle cover 60 will not normally deploy as the front end of the autoinjector 1 is still being held in contact with the injection site.

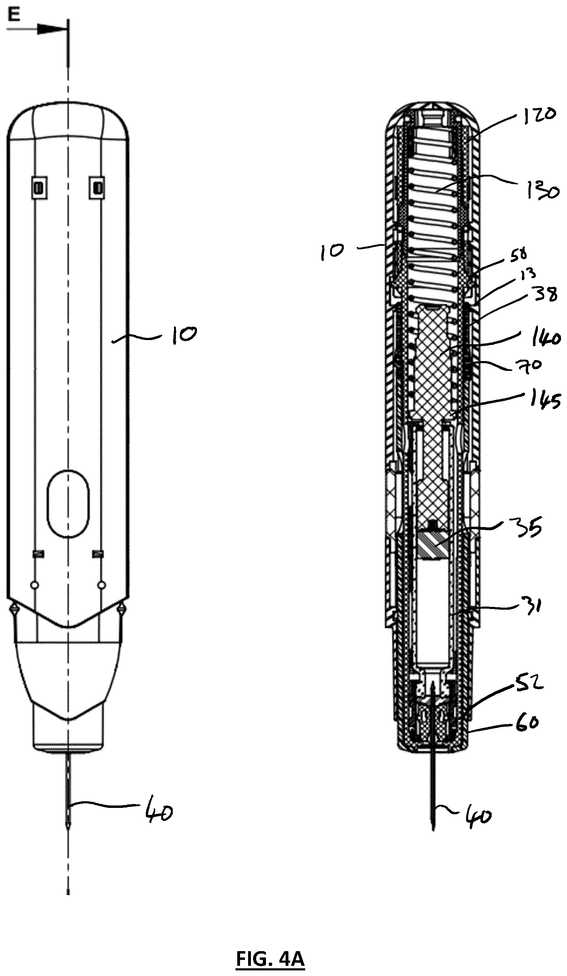

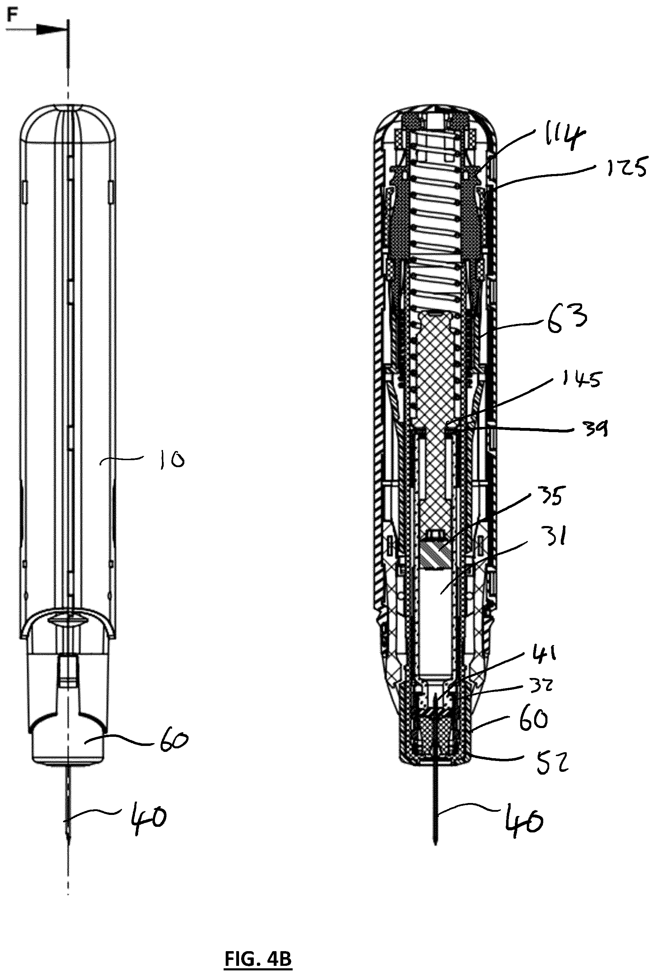

As shown in Figure 4, following release of the piston rod 140, the spring 130 urges the cartridge assembly 30 forward. Initially, this causes the forward end of the needle to be pushed through the porous filtration membrane 54. When the needle guide 42 reaches the internal front surface of the forward end of the cartridge container 52, the rearward end of the needle will pass through the septum 32a of the cartridge 30 to establish a fluid pathway after the needle has been fully extended. Once the needle assembly 40 and cartridge 31 reach the front of the cartridge container 52, they can no longer move and the force of the spring 130 causes the piston rod 140 to move the stopper 35 forward in the cartridge 31 and administer the dose via the needle 40. When a full dose has been administered, the flange 145 of the piston rod 140 reaches the lip 39 of the cartridge sleeve 38 and prevents further forward movement. Thus, it will be appreciated that the position of the flange 145 on the piston rod 140 provides a convenient feature which can be modified to provide autoinjectors able to deliver different volumes and thus different doses of a medicament.

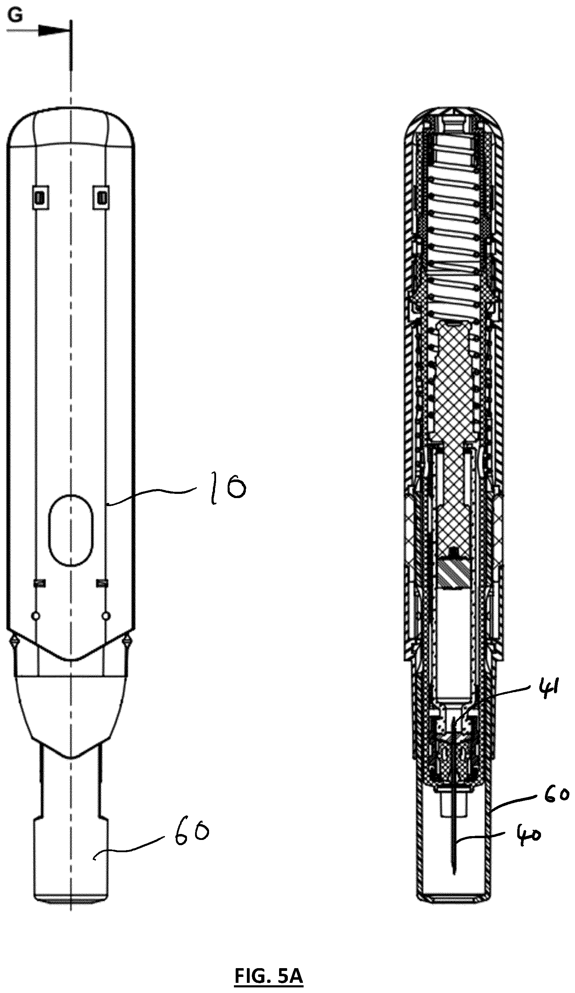

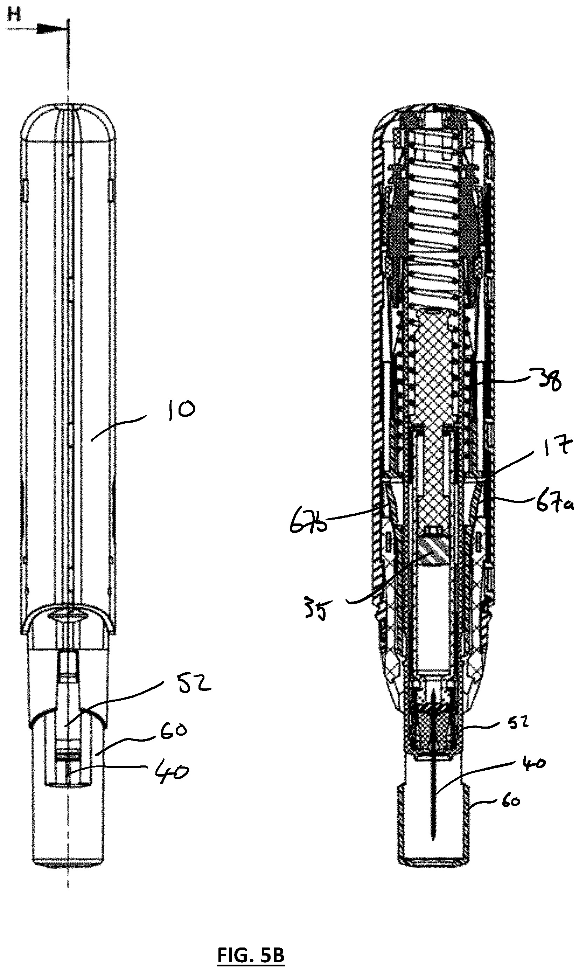

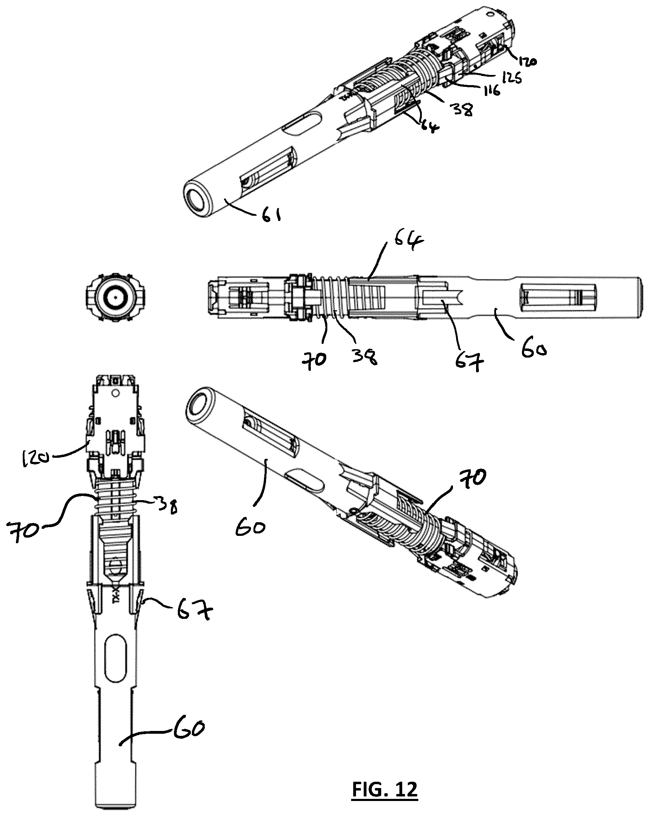

As shown in Figures 5 and 12, once the injection is complete the user can remove the autoinjector 1 from the injection site. As the needle cover 60 was unlocked during activation and throughout the injection phase, the needle cover spring 70 can now act on the needle cover 60 and deploy the needle cover out of the forward end of the main casing 10, unless the front-end of the autoinjector is held and forced manually to not deploy When the needle cover 60 has fully deployed (it has extended sufficiently that the forward end of the cover is beyond the forwardmost tip of the needle), the second locking assembly acts to lock the cover. The second locking assembly acts automatically as a result of the outwardly extending resilient members 67 of the needle cover 60 passing over inwardly extending ribs 17 formed in the main casing 10. The angled arrangement of the resilient members 67 enables them to freely cam over the ribs 17 during forward movement of the cover 60 (under the force of the cover spring 70). Once beyond the rib 17, the resilient members 67 spring back to their outward position. The needle cover 60 is, therefore, locked against rearward movement as the resilient members will not pass over the ribs 17 in the reverse direction.

In this configuration, the autoinjector 1 cannot be re-activated and is safe since the needle 40 is fully shrouded by the cover 60. The user can visually see that the autoinjector is no longer active due to the position of the needle cover and because the window 15 of the main casing 10 is now blocked by a portion of the needle cover. Due to the forward extension of the needle cover 60 the user cannot replace the cap 20 on the main casing 10 (as the detent features 19, 23 of the main casing 19 and cap 20 cannot be brought into alignment). It may also be noted that the cross-sectional profile of the needle cover 60 is circular whereas the cap 20 and main casing 10 are both oval. This provides a useful visual indication to discourage a user from attempting to recap a used device. Further, the geometry of the cap 20 and main casing 10 can be selected to ensure that should recapping be attempted (prior to activation) the cap will not place pressure on the needle cover (as it will only engage the larger oval profile of the main casing). After use, the needle cover 60 extends so far beyond the main casing 10 that recapping is not possible.

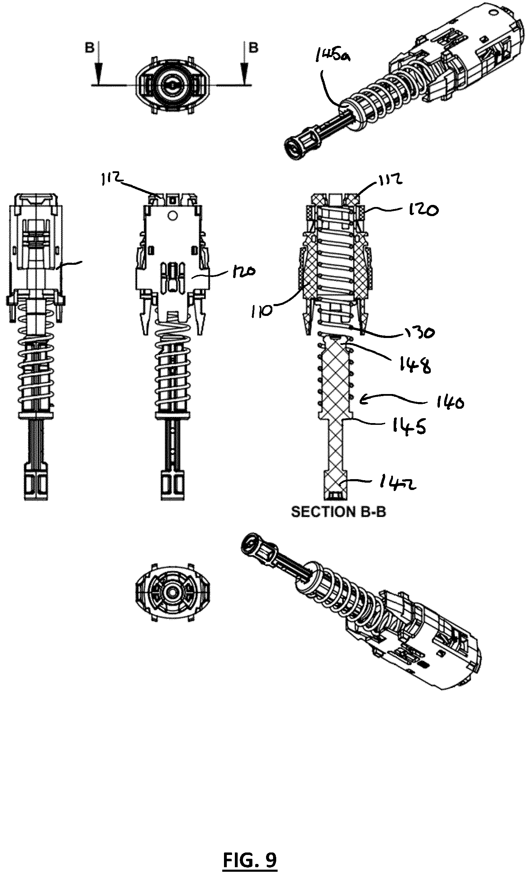

Figure 14A to 14D illustrate how autoinjectors of embodiments can be adapted to provide a variety of delivery doses by selection of an appropriate piston rod 140 and without the need to modify other aspects of the device. The piston rod 140 of figure 14A is as shown in the embodiments of figures 1 to 12 and includes a single flange 145' in a central axial location which defines a separation between the forward 141 and rearward 146 portions of the piston rod. The rearward face of the flange 145a provides a spring shoulder against which a forward end of the spring 130 is mounted. The forward face 145b of the flange 145 defines a stop surface which abuts the lip 39 of the cartridge sleeve 38 to prevents further forward movement of the piston rod relative to the cartridge 31 during drug delivery. In figure 14B the piston rod 140' has an increased axial spacing "x" between the forward face 145b' and the rearward face 145a'. In the illustrated arrangement the additional axial spacing is provided by having two parallel spaced apart flange members 145' - it will however be appreciated that a solid flange of increased axial thickness could have the same function. The rearward face 145a' can be positioned in the same axial location as in the embodiment of fig 14A such that the action of the spring 130 is unaltered by the use of the modified piston rod 140'. In contrast the forward face 145b' being positioned axial forward ensures that the extent to which the piston rod 140 can enter the cartridge 31 before being stopped by engagement between the face 145a' and the lip 39 is reduced. This the delivery dose provided by actuation of the autoinjector is reduced. Further variants are shown in figures 14C and 14D which are substantially identical to the embodiment of figure 14B but with increased axial spacing "y" and "z" is stopped between the respective rearward face 145a" 145a‴ and the respective forward face 145b" 145b"'. The greater the axial spacing the lower the dose that will be delivered upon actuation of the device - this enables a range of devices to be manufactured which deliver different doses using the same main components with only the piston rod changed (for example enabling a single production line to produce all the injectors).

As shown in Figures 19A to 19D it may be noted that the autoinjector 1 is typically provided to the end user with a label 200 wrapped (and bonded) around the outer surface of the main casing 10. In the initial assembled configuration shown in Figure 19A the autoinjector 1 does not have a label. In this arrangement it will be noted that a number of apertures exist in the main casing 10. For example, apertures 151 at the rearward end of the casing 10 may include at least one opening provided for the safety pin 150 which prevents operation of the power pack during manufacturing. Further apertures 155 may be provided at the forward end of the casing 10 and may, for example, be provided to assist the flow of gas into the needle compartment S during sterilisation of the autoinjector. The label 200 may be formed as a single net shape as shown in fig 19C. The label has opposing sides 205, 206, a straight end 208 and a profiled forward end 210 to match the shape and configuration of the forward end of the autoinjector casing 10. A pair of cut-outs 201, 202 are included and are shaped and sized to match the windows 15 of the casing 10. As seen in figure 19D the label can be rolled to form a tubular shape with the sides 205 and 206 adjacent and axially extending. In the rolled configuration the cut-outs 201, 202 are diametrically opposed. As shown in Figure 19B, as a final step of the assembly of the autoinjector 1, the label 200 is wrapped around the casing 10 (and may for example be self-adhesive to bond the label to the surface of the casing). With the label 200 in place, the apertures 151, 155 of the casing 10 are covered and, as such, the label 200 can provide ingress protection to the autoinjector 1. As the main casing 10 is formed of two parts, the label 200 may also be arranged to cover the join between the casing parts (which could for example provide tamper evident protection and enhanced robustness).

Although aspects of the invention have been described above with reference to a preferred embodiment, it will be appreciated that various changes or modification may be made without departing from the scope of the invention as defined in the appended claims.

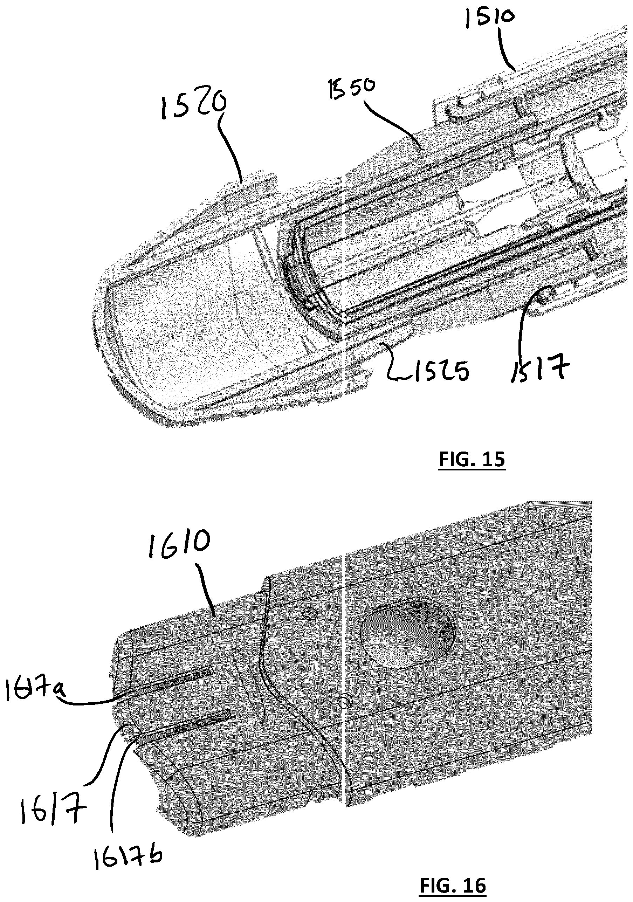

For example, it may be appreciated that the locking arrangement between the actuation unit 50 and the cap 20 can be formed in a variety of ways. One alternate configuration is shown in the embodiment of figure 15. In this arrangement the cap 1520 is formed with rearwardly projecting ribs 1525 which force or hold locking arms 1517 in an outwardly direction to engage with the main casing 1510. When the locking arms 1517 are inwardly supported by the ribs 1525 they cannot inwardly compress to ride over an inwardly projecting shoulder (or other such feature) of the housing. As such, the cap 1520 blocks relative movement between the housing 1510 and the actuation unit 1550.

Another embodiment is shown in Figure 16 in which locking arms 1617 are integrally formed as part of the main casing 1610. The locking arm 1617 can be formed as a forwardly extending tongue which is separated from the adj acent portion of the housing by axially extending slots 1617a and 1617b so that the forward end is resiliently deflectable. The cap in this embodiment may then act against this forwardly extending tongue in the same manner as the locking arm 17 of the embodiment of figures 1 to 14.

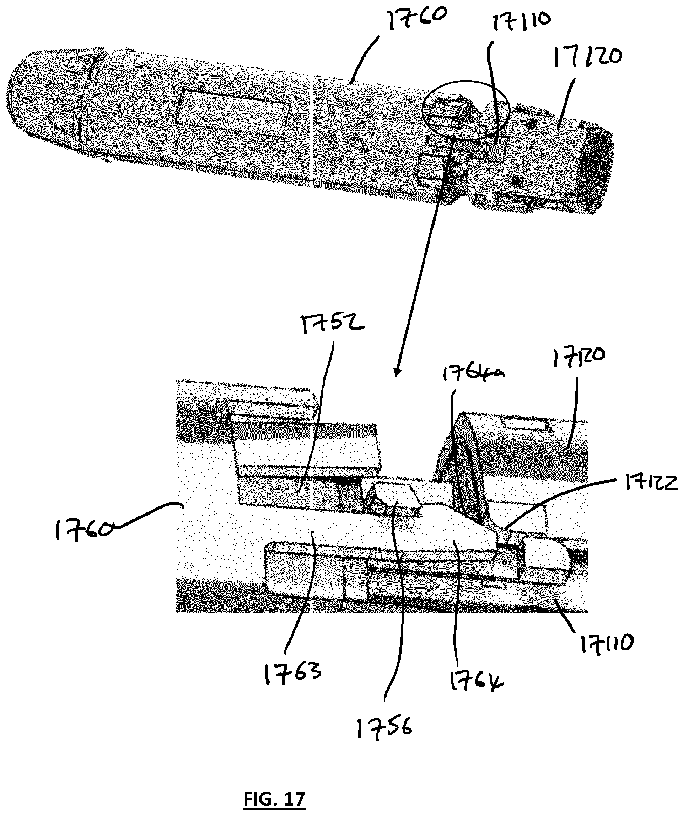

Fig 17 shows an alternate arrangement for the first locking assembly which retains the needle cover in its first (retracted) position. In this embodiment the hooks 1764 are radially outwardly directed and provided on a pair of arms 1763 which are formed close to the centreline of the rear portion of the needle cover 1760. An identical arrangement may typically be provided on the opposite side of the autoinjector. The hooks 1764 engage bosses 1756 to lock the needle cover 1760 in position. It may be noted that the bosses 1756 in this embodiment are formed on the inner body 17110 rather than the cartridge container 1752 (and, as the cartridge container 1752 and inner body 17110 are fixed relative to each other, either component may include the bosses).

The outer body 17120 of the power unit 17100 includes cooperating cam surfaces 17122 which face angled surfaces 1764a formed on the hooks 1764. In use, it will be appreciated that relative movement between the needle cover 1760 and the outer body 17120 (i.e. during actuation of the autoinjector) will cause the angled surfaces 1764a to be engaged by the cam surfaces 17122. The cam surfaces 17122 squeeze the arms 1763 together and release the hooks 1764 from engagement with the bosses 1756 to free the needle cover 1760 for forward movement.

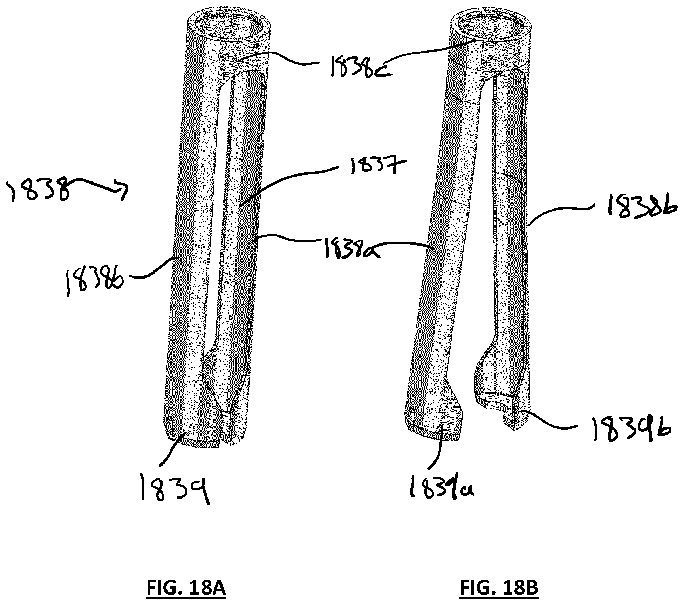

Figure 18A and 18B show an alternate a cartridge sleeve 1838 which may be used in embodiments. Figure 18A shows the in-use configuration of the cartridge sleeve 1838 (i.e. when assembled inside the autoinjector) and figure 18B shows a pre-assembly configuration. In contrast to the embodiment of Figures 1 to 14 which is open on one side (with the exception of the lip 39) to allow insertion of the cartridge 31, the cartridge sleeve 1838 is formed of two axially extending leg potions 1838a and 1838b which are connected at the forward end by an annular portion 1838c. The sides of the leg portions 1838a and 1838b are profiled to define a window 1837 therebetween when positioned around a cartridge. The lip 1839 is formed by respective portions 1839a and 1839b at the rear end of the cartridge sleeve 1838 and will seat on the rear of the cartridge as with the lip 39 of the previous embodiments. The cartridge sleeve may have an initial configuration in which the legs 1838a and 1838b are splayed, to allow ease of insertion of a cartridge 31, as shown in figure 18B and in use the legs may be resiliently deflected together to close around cartridge (and will be held in place by the surrounding cartridge holder 52). Alternatively, the at rest configuration of the sleeve 1838 may be as shown in figure 18A and the legs 1838a and 1838b may be resiliently splayed to insert the cartridge 31 before springing back into the closed configuration.

## Claims

1. An autoinjector (1), comprising:
a main casing (10);
a cartridge assembly (30) disposed within the main casing and including a cartridge (31) and a needle (40), the cartridge containing a medicament;
a power pack (100) disposed within the main casing, the power pack for moving the cartridge and the needle, relative to the main casing, from a storage position to an injection position when actuated;
a cartridge container (52) and a needle cover (60) disposed between the cartridge container and the main casing; and
a safety cap (20);
**characterized in that**
the autoinjector comprises an actuation unit (50) comprising the cartridge container and the needle cover, the actuation unit operably coupled to the power pack and retractable, relative to the main casing, from a first position to an actuation position for actuating the power pack and thereafter the needle cover is extendable to an extended position covering the needle when the cartridge and the needle are in the injection position;
wherein the safety cap is removable from a mounted position on a front end of the main casing to expose a front portion of the actuation unit, the safety cap arranged to press a locking arm (17a, 17b) against the actuation unit when the safety cap is in the mounted position to lock the actuation unit against retraction from the first position until the safety cap is removed;
wherein the needle cover includes a through opening (66a, 66b) and the cartridge container includes a recess (57a, 57b) so that a front end portion of the locking arm extends through the opening and is pressed by the safety cap into engagement with the recess of the cartridge container.

2. The autoinjector of Claim 1, wherein the safety cap includes a rib (25a, 25b) or other inwardly projecting structure for pressing the locking arm against the actuation unit to lock the actuation unit against retraction from the first position until the safety cap is removed.

3. The autoinjector of Claim 1, wherein the actuation unit and the locking arm are lockingly engaged when the safety cap is in the mounted position and are free or biased to disengage from locking when the safety cap is removed.

4. The autoinjector of Claim 1, wherein the cartridge container includes the recess in an outer surface thereof and the locking arm is pressed into engagement with the recess by the safety cap when the safety cap is in the mounted position.

5. The autoinjector of Claim 4, wherein the locking arm is free or biased to splay outwardly out of engagement with the recess when the safety cap is removed.

6. The autoinjector of Claim 1, wherein the front portion of the actuation unit has a circular cross-section and the safety cap has an oval cross-section.

7. The autoinjector of Claim 1, wherein the safety cap and the main casing include mechanically cooperating features including a detent (19, 23) to releasably mount the safety cap on the front end of the main housing.

8. The autoinjector of Claim 1, wherein the safety cap and the front end of the main casing have cooperating oval cross-sections to provide for a slide fit mounting and recapping of the safety cap and the front end of the main casing.

9. The autoinjector of Claim 1, wherein the safety cap is removable from the front end of the main casing by translation without rotation.

10. The autoinjector of Claim 1, wherein the safety cap is recappable.

11. The autoinjector of Claim 1, comprising first and second locking arms that are arranged at diametrically opposed positions.

12. The autoinjector of claim 11, wherein the safety cap is arranged to press the first and second locking arms against the respective recesses of the cartridge container and extend through respective through openings of the needle cover when the safety cap is in the mounted position to lock the actuation unit against retraction from the first position until the safety cap is removed.

13. The autoinjector of Claim 1, wherein the locking arm has a front end that is enlarged to project inwardly.

14. The autoinjector of Claim 1, wherein the main casing comprises a window opening and further comprises a transparent window piece (16) inserted into the main casing to close the window opening, and wherein a forwardly projecting locking arm (17a, 17b) is formed integrally with the window piece optionally, wherein the main casing comprises a pair of diametrically opposed window openings and a transparent window piece is inserted into each window opening and wherein each window piece comprises a forwardly projecting locking arm.

15. The autoinjector of Claim 1, wherein the actuation unit includes a sterile chamber (S) for the needle and removal of the safety cap does not affect sterility of the sterile chamber.

16. The autoinjector of Claim 1, wherein the medicament is epinephrine.

## Patentansprüche

1. Autoinjektor (1), umfassend:
ein Hauptgehäuse (10);
eine Kartuschenbaugruppe (30), die innerhalb des Hauptgehäuses angeordnet ist und eine Kartusche (31) und eine Nadel (40) beinhaltet, wobei die Kartusche ein Medikament enthält;
ein Antriebsmodul (100), das innerhalb des Hauptgehäuses angeordnet ist und dazu dient, die Kartusche und die Nadel bei Betätigung relativ zum Hauptgehäuse aus einer Aufbewahrungsposition in eine Injektionsposition zu bewegen;
einen Kartuschen-Behälter (52) und eine Nadel-Abdeckung (60), die zwischen dem Kartuschen-Behälter und dem Hauptgehäuse angeordnet sind; und
eine Sicherheitskappe (20);
**dadurch gekennzeichnet, dass**
der Autoinjektor eine Betätigungseinheit (50) umfasst, die den Kartuschenbehälter und die Nadelabdeckung umfasst, wobei die Betätigungseinheit funktionsfähig mit dem Antriebspaket gekoppelt und relativ zum Hauptgehäuse aus einer ersten Position in eine Betätigungsposition zum Betätigen des Antriebspakets zurückziehbar ist und danach die Nadelabdeckung in eine ausgefahrene Position ausgefahren werden kann, in der sie die Nadel abdeckt, wenn sich die Kartusche und die Nadel in der Injektionsposition befinden;
wobei die Sicherheitskappe aus einer montierten Position an einem vorderen Ende des Hauptgehäuses entfernbar ist, um einen vorderen Abschnitt der Betätigungseinheit freizulegen,
wobei die Sicherheitskappe so angeordnet ist, dass sie einen Sicherungsarm (17a, 17b) gegen die Betätigungseinheit drückt, wenn sich die Sicherheitskappe in der montierten Position befindet, um die Betätigungseinheit gegen ein Zurückziehen aus der ersten Position zu sichern, bis die Sicherheitskappe entfernt wird;
wobei die Nadelabdeckung eine Durchgangsöffnung (66a, 66b) beinhaltet und der Kartuschenbehälter eine Aussparung (57a, 57b) beinhaltet, so dass ein vorderer Endabschnitt des Sicherungsarms durch die Öffnung ragt und durch die Sicherheitskappe in Eingriff mit der Aussparung des Kartuschenbehälters gedrückt wird.

2. Autoinjektor nach Anspruch 1, wobei die Sicherheitskappe eine Rippe (25a, 25b) oder eine andere einwärts gerichtete Struktur beinhaltet, um den Sicherungsarm gegen die Betätigungseinheit zu drücken und die Betätigungseinheit gegen ein Zurückziehen aus der ersten Position zu sichern, bis die Sicherheitskappe entfernt wird.

3. Autoinjektor nach Anspruch 1, wobei die Betätigungseinheit und der Sicherungsarm ineinander greifen, wenn sich die Sicherheitskappe in der montierten Position befindet, und frei oder vorspannt sind, um sich aus der Verriegelung zu lösen, wenn die Sicherheitskappe entfernt wird.

4. Autoinjektor nach Anspruch 1, wobei der Kartuschenbehälter die Aussparung in seiner Außenfläche beinhaltet und der Sicherungsarm durch die Sicherheitskappe in Eingriff mit der Aussparung gedrückt wird, wenn sich die Sicherheitskappe in der montierten Position befindet.

5. Autoinjektor nach Anspruch 4, wobei der Sicherungsarm frei oder vorspannt ist, um sich auswärts gerichtet aus dem Eingriff mit der Aussparung zu bewegen, wenn die Sicherheitskappe entfernt wird.

6. Autoinjektor nach Anspruch 1, wobei der vordere Abschnitt der Betätigungseinheit einen kreisförmigen Querschnitt aufweist und die Sicherheitskappe einen ovalen Querschnitt aufweist.

7. Autoinjektor nach Anspruch 1, wobei die Sicherheitskappe und das Hauptgehäuse mechanisch zusammenwirkende Merkmale beinhalten, einschließlich einer Arretierung (19, 23), um die Sicherheitskappe lösbar am vorderen Ende des Hauptgehäuses anzubringen.

8. Autoinjektor nach Anspruch 1, wobei die Sicherheitskappe und das vordere Ende des Hauptgehäuses zusammenwirkende ovale Querschnitte aufweisen, um eine Gleitpassung zum Anbringen und Wiederaufsetzen der Sicherheitskappe und des vorderen Endes des Hauptgehäuses bereitzustellen.

9. Autoinjektor nach Anspruch 1, wobei die Sicherheitskappe entfernbar durch Verschieben ohne Drehung vom vorderen Ende des Hauptgehäuses ist.

10. Autoinjektor nach Anspruch 1, wobei die Sicherheitskappe wiederaufsetzbar ist.

11. Autoinjektor nach Anspruch 1, umfassend einen ersten und einen zweiten Sicherungsarm, die
an diametral gegenüberliegenden Positionen angeordnet sind.

12. Autoinjektor nach Anspruch 11, wobei die Sicherheitskappe so angeordnet ist, dass sie den ersten und den zweiten Sicherungsarm gegen die jeweiligen Aussparungen des Kartuschenbehälters drückt und sich durch die jeweiligen Durchgangsöffnungen der Nadelabdeckung erstreckt, wenn sich die Sicherheitskappe in der montierten Position befindet, um die Betätigungseinheit gegen ein Zurückziehen aus der ersten Position zu sichern, bis die Sicherheitskappe entfernt wird.

13. Autoinjektor nach Anspruch 1, wobei der Sicherungsarm ein vorderes Ende aufweist, das vergrößert ist, um einwärts gerichtet zu stehen.

14. Autoinjektor nach Anspruch 1, wobei das Hauptgehäuse eine Fensteröffnung umfasst und ferner ein transparentes Fensterstück (16) umfasst, das in das Hauptgehäuse eingesetzt ist, um die Fensteröffnung zu verschließen, und wobei ein nach vorne vorstehender Sicherungsarm (17a, 17b) optional einstückig mit dem Fensterteil gebildet ist, wobei das Hauptgehäuse ein Paar diametral gegenüberliegender Fensteröffnungen umfasst und ein transparentes Fensterteil in jede Fensteröffnung eingesetzt ist und wobei jedes Fensterteil einen nach vorne ragenden Sicherungsarm umfasst.

15. Autoinjektor nach Anspruch 1, wobei die Betätigungseinheit eine sterile Kammer (S) für die Nadel beinhaltet und das Entfernen der Sicherheitskappe die Sterilität der sterilen Kammer nicht beeinträchtigt.

16. Autoinjektor nach Anspruch 1, wobei das Medikament Adrenalin ist.

## Revendications

1. Auto-injecteur (1), comprenant :
un boîtier principal (10) ;
un ensemble cartouche (30) disposé à l'intérieur du boîtier principal et comportant une cartouche (31) et une aiguille (40), la cartouche contenant un médicament ;
un bloc d'alimentation (100) disposé à l'intérieur du boîtier principal, le bloc d'alimentation étant destiné à déplacer la cartouche et l'aiguille, par rapport au boîtier principal, d'une position de stockage à une position d'injection, lorsqu'il est actionné ;
un récipient de cartouche (52) et un couvercle d'aiguille (60) disposé entre le récipient de cartouche et le boîtier principal ; et
un capuchon de sécurité (20) ;
**caractérisé en ce que**
l'auto-injecteur comprend une unité d'actionnement (50) comprenant le récipient de cartouche et le couvercle d'aiguille, l'unité d'actionnement étant couplée de manière fonctionnelle au bloc d'alimentation et rétractable, par rapport au boîtier principal, d'une première position à une position d'actionnement pour actionner le bloc d'alimentation et, par la suite, le couvercle d'aiguille est extensible jusqu'à une position étendue recouvrant l'aiguille lorsque la cartouche et l'aiguille sont dans la position d'injection ;
dans lequel le capuchon de sécurité peut être retiré d'une position montée sur une extrémité avant du boîtier principal pour exposer une partie avant de l'unité d'actionnement, le capuchon de sécurité étant disposé pour presser un bras de verrouillage (17a, 17b) contre l'unité d'actionnement lorsque le capuchon de sécurité est dans la position montée pour verrouiller l'unité d'actionnement contre la rétraction de la première position jusqu'à ce que le capuchon de sécurité soit retiré ;
dans lequel le couvercle d'aiguille comporte une ouverture traversante (66a, 66b) et le récipient de cartouche comporte un évidement (57a, 57b) de sorte qu'une partie d'extrémité avant du bras de verrouillage s'étend à travers l'ouverture et est pressée par le capuchon de sécurité en prise avec l'évidement du récipient de cartouche.

2. Auto-injecteur selon la revendication 1, dans lequel le capuchon de sécurité comporte une nervure (25a, 25b) ou une autre structure faisant saillie vers l'intérieur pour presser le bras de verrouillage contre l'unité d'actionnement pour verrouiller l'unité d'actionnement contre la rétraction de la première position jusqu'à ce que le capuchon de sécurité soit retiré.

3. Auto-injecteur selon la revendication 1, dans lequel l'unité d'actionnement et le bras de verrouillage sont en prise de manière verrouillée lorsque le capuchon de sécurité est dans la position montée et sont libres ou sollicités pour se désengager du verrouillage lorsque le capuchon de sécurité est retiré.

4. Auto-injecteur selon la revendication 1, dans lequel le récipient de cartouche comporte l'évidement dans une surface externe de celui-ci et le bras de verrouillage est pressé pour se mettre en prise avec l'évidement par le capuchon de sécurité lorsque le capuchon de sécurité est dans la position montée.

5. Auto-injecteur selon la revendication 4, dans lequel le bras de verrouillage est libre ou sollicité pour s'écarter vers l'extérieur hors de prise avec l'évidement lorsque le capuchon de sécurité est retiré.

6. Auto-injecteur selon la revendication 1, dans lequel la partie avant de l'unité d'actionnement présente une section transversale circulaire et le capuchon de sécurité présente une section transversale ovale.

7. Auto-injecteur selon la revendication 1, dans lequel le capuchon de sécurité et le boîtier principal comportent des structures coopérants mécaniquement comportant une détente (19, 23) pour monter le capuchon de sécurité de manière amovible sur l'extrémité avant du boîtier principal.

8. Auto-injecteur selon la revendication 1, dans lequel le capuchon de sécurité et l'extrémité avant du boîtier principal présentent des sections transversales ovales coopérantes pour permettre un montage ajusté coulissant et un replacement du capuchon de sécurité et de l'extrémité avant du boîtier principal.

9. Auto-injecteur selon la revendication 1, dans lequel le capuchon de sécurité peut être retiré de l'extrémité avant du boîtier principal par translation sans rotation.

10. Auto-injecteur selon la revendication 1, dans lequel le capuchon de sécurité peut être replacé.

11. Auto-injecteur selon la revendication 1, comprenant des premier et deuxième bras de verrouillage qui sont disposés à des positions diamétralement opposées.

12. Auto-injecteur selon la revendication 11, dans lequel le capuchon de sécurité est disposé de manière à presser les premier et deuxième bras de verrouillage contre les évidements respectifs du récipient de cartouche et s'étendre à travers des ouvertures traversantes respectives du couvercle d'aiguille lorsque le capuchon de sécurité est dans la position montée pour verrouiller l'unité d'actionnement contre la rétraction de la première position jusqu'à ce que le capuchon de sécurité soit retiré.

13. Auto-injecteur selon la revendication 1, dans lequel le bras de verrouillage présente une extrémité avant qui est élargie de manière à faire saillie vers l'intérieur.

14. Auto-injecteur selon la revendication 1, dans lequel le boîtier principal comprend une ouverture de fenêtre et comprend en outre une pièce fenêtre transparente (16) insérée dans le boîtier principal pour fermer l'ouverture de fenêtre, et dans lequel, optionnellement, un bras de verrouillage faisant saillie vers l'avant (17a, 17b) est formé d'un seul tenant avec la pièce fenêtre, dans lequel le boîtier principal comprend une paire d'ouvertures de fenêtre diamétralement opposées et une pièce fenêtre transparente est insérée dans chaque ouverture de fenêtre et dans lequel chaque pièce fenêtre comprend un bras de verrouillage faisant saillie vers l'avant.

15. Auto-injecteur selon la revendication 1, dans lequel l'unité d'actionnement comporte une chambre stérile (S) pour l'aiguille et le retrait du capuchon de sécurité n'affecte pas la stérilité de la chambre stérile.

16. Auto-injecteur selon la revendication 1, dans lequel le médicament est de l'épinéphrine.
